# EUROPEAN PATENT SPECIFICATION

(11) **EP 3 988 158 B1**
(45) Date of publication and mention of the grant of the patent: **04.12.2024**
(21) Application number: 20203944.2
(22) Date of filing: 26.10.2020
(51) Int. Cl.: A61N 1/02, A61N 1/372, G16H 20/00, H04W 40/00, H04W 12/00

(54) **SYSTEM FOR PLANNING AND/OR CONTROLLING NEUROMODULATION**
SYSTEM ZUR PLANUNG UND/ODER STEUERUNG VON NEUROMODULATION
SYSTÈME DE PLANIFICATION ET/OU DE CONTRÔLE D'UNE NEUROMODULATION

(43) Date of publication of application: 27.04.2022
(73) Proprietor: ONWARD Medical N.V., 5611 ZX Eindhoven (NL)
(72) Inventor: BROUNS, Robin, 5656 AE Eindhoven (NL); DELATTRE, Vincent, 5656 AE Eindhoven (NL)
(74) Representative: DTS Patent- und Rechtsanwälte PartmbB

(56) References cited:
- WO-A1-02/24064
- US-A1- 2008 097 551
- US-A1- 2009 024 178

## Description

The present invention relates to a system for planning and/or controlling neuromodulation. Further, the present invention relates to a method for planning neuromodulation.

Neuromodulation systems typically comprise at least a medical device for applying neuromodulation, a controller and at least one programmer.

Medical professionals (e.g. therapists, nurses, physiotherapists, clinicians) and patients typically communicate with a medical device using an electronic programmer, in particular a clinician programmer (e.g. space time programmer) and a patient programmer respectively. Each of these programmers can be a handheld device. A clinician programmer allows a medical professional to define the particular electrical stimulation therapy to be delivered to a target area of the patient's body, while a patient programmer allows e.g. a patient or his/her relatives, to alter one or more parameters of the electrical stimulation therapy and/or start and/or stop stimulation and/or monitor properties of the neuromodulation system or a patient's health metrics (e.g. physiological data while receiving electrical stimulation therapy).

US7885712B2 discloses techniques for increasing the safety of medical device programming using general purpose hardware, such as a general-purpose personal computer. In some embodiments, a system includes an intermediate computing device comprising an applications module. Information from the applications module, such as instructions for an implantable medical device (IMD), may be presented to a user via a user input terminal that is separate from the intermediate computing device. A user may interact with the user input terminal to select an instruction from the applications module, and the intermediate computing device may transmit the selected instruction to the IMD. In some embodiments, the intermediate computing device comprises a web server and the user input terminal comprises a web browser configured to access the web server. In other embodiments, the intermediate computing device comprises a client server and the user input terminal comprises a client.

US2009/024178A1 discloses system for remote programming of a programmable personal medical device, in particular an implantable medical device such as a cardiac pacemaker, defibrillator, or the like. A patient intermediary device may be positioned in proximity to the patient and thus in proximity to the implantable medical device. A programming device is located at a distance from the patient and from the patient intermediary device. The programming device and the patient intermediary device are tuned to the implantable medical device, at least for the duration of the remote programming. The patient intermediary device is equipped with a first interface and a second interface that are designed for a transmission of a data set from the programming device to the patient intermediary device. The patient intermediary device receives the data set, and an authentication unit verifies the data set for the presence of a valid authentication. The data set including the programming instruction is only transmitted to the implantable medical device when the presence of a valid authentication in the data set is confirmed by the authentication unit. The object of the present invention is to provide an alternative system for planning and/or controlling and/or programming neuromodulation, wherein the system ensures secure and controlled data transfer.

This object is solved according to the present invention with a system for planning and/or controlling neuromodulation, with the features of claim 1. Accordingly, a system for planning and/or controlling neuromodulation, at least comprising:
a medical device,
an intermediate computing device,
a first user input terminal,
a second user input terminal,
wherein the medical device, the intermediate computing device, the first user input terminal and/or the second user input terminal are at least temporarily connected,
wherein the system further comprises at least one connection managing module, wherein the at least one connection managing module is configured and arranged for
   - allowing only unidirectional connection and/or unidirectional data transfer between the intermediate computing device and the first user input terminal, the intermediate computing device and the second user input terminal and/or the first user input terminal and the second user input terminal,
   - providing only connection and/or data transfer between the intermediate computing device and the first user input terminal and/or the intermediate computing device and the second user input terminal when there is no connection and/or data transfer between the first user input terminal and the second user input terminal.

The invention is based on the basic idea that in the context of neuromodulation, especially neurostimulation, stimulation parameters can be reliably planned and/or controlled with said system, wherein precise stimulation protocols are enabled by avoiding data interference, in particular by means of the connection managing module. In particular, interference of data and/or signals transmitted between one user input terminal and the other user input terminal and/or the intermediate computing device is avoided and/or excluded by avoiding bidirectional connection and/or data transfer between these units. In other words, unidirectional communication channels are provided. In other words, one device, module or terminal may send data and/or instructions in one direction to another device, module or terminal. Therefore, controlled and secure transmission of data and/or signals at all time while the system is operating is enabled. Further, encryption of data may be enabled. Further, reduced energy consumption of the system and prolonged battery life may be achieved through unidirectional data transfer and/or selective temporal connections.

Data interference may occur when transmissions are sent from a transmitter (device, module or terminal) to a receiver (other device, module or terminal) and when transmissions are simultaneously sent from said receiver (which is then also a transmitter) to the transmitter (which is then also a receiver). Also, data interference may occur within a system when transmissions are sent from a transmitter (device, module or terminal) to a receiver (other device, module or terminal) and when transmissions are simultaneously sent from another transmitter to another or the same receiver.

Data interference may alternatively and/or additionally occur when data are stored in at least one unit of the system (e.g. intermediate computing device, first user input terminal, second user input terminal, medical device or controller). In particular, the data stored on it may be newer or older than what is stored in another, connected device (other element of the system). In particular, interference of data may then be avoided and/or excluded by adapting the data stored in both units to the newer version and/or avoiding simultaneous data transfer between each of these units with a third unit.

In particular, the intermediate computing device may enable doing sanity-checks on commands received from the first user input terminal and/or the second user input terminal, preventing that the first user input terminal and/or the second user input terminal send invalid commands directly into the medical device. In particular, the intermediate computing device may comprise and/or use a security system that monitors and/or controls incoming and/or outcoming signal traffic based on predetermined security rules, such as an unauthorized access block method, e.g. 'a firewall'. This may, for instance, enable secure data transfer.

In one embodiment, the at least one connection managing module may be embodied as software module, in particular in the intermediate computing device. Additionally, or alternatively, the connection managing module may be embodied as software module in the first user input terminal. Additionally, or alternatively, the connection managing module can be embodied in the second user input terminal. Alternatively, the connection managing module may be embodied in another device comprised within or linked to the system.

Alternatively, the at least one connection managing module may be or may comprise a communication controller, in particular an electrical communication controller.

The medical device may be a neuromodulator, in particular a neurostimulator. Also, the medical device may be a battery powered device. The medical device may be implanted. In a preferred embodiment, the medical device is or comprises a pulse generator, in particular an implantable pulse generator (IPG), which comprises or is linked to one or multiple stimulation leads, each with at least one electrode. Its intended use is to deliver electrical stimulation via the at least one lead to at least one stimulation site of a patient equipped with the system based on commands (neuromodulation control data) received from the intermediate computing device.

The intermediate computing device may be or may comprise a controller. In particular, the intermediate computing device may be a body worn-platform (directly or indirectly) to execute the control software. In particular, in a specific embodiment, the intermediate computing device can be implanted.

The intermediate computing device processes data that is acquired e.g. from the first user input terminal, the second user input terminal and/or the medical device and programs the medical device to deliver correct (planned) stimulation. In other words, the intermediate computing device is configured and arranged to provide neuromodulation control data to the medical device, in particular neuromodulation control data for at last one specific task (e.g. an activity training/exercise/muscular response/ autonomic system response/sphincter response/blood pressure response) and/or at least one specific neuromodulation therapy (e.g. to restore motoric function, autonomic function, blood pressure (hypotension, hypertension), bladder control, bowel control, sphincter control and/or sexual function), at least partially on the basis of the information obtained from the first user input terminal and/or the second user input terminal.

The first user input terminal, in some embodiments also called the patient programmer (PP) may inter alia allow the user, e.g. the patient or a relative of the patient, to select a specific stimulation program and/or input therapy parameter (e.g. for a specific task or a specific medical indication) and/or to alter one or more parameters of the stimulation therapy program and/or to start and/or stop and/or ramp up and/or ramp down and/or modify and/or pause stimulation and/or to monitor properties of the neuromodulation system or at least one of patient's health metrics (e.g. physiological data while receiving electrical stimulation therapy).

In particular, the first user input terminal may comprise and/or may offer a list of pre-installed (simplified) control commands to be selected from and/or altered by the user, e.g. the patient or a relative of the patient. In particular, by selecting and/or altering at least one of a pre-installed control commands simple handling of the system is enabled for the user.

In one embodiment, the first user input terminal is capable to receive data from the intermediate computing device or the second user input terminal, display data, receive input from the user (e.g. patient), send it back to the intermediate computing device or the second user input terminal. In other words, the first user input terminal can receive, display, process and/or resend data.

The second user input terminal, or also called the clinician programmer, can inter alia be used to receive inter alia stimulation parameters, patient data, physiological data, training data etc. and/or for defining and/or altering stimulation parameters (inter alia electrode configuration, frequency, pulse width, amplitude) and/or allowing adjustment(s) to the stimulation. The second user input terminal may additionally and/or alternatively read and/or monitor properties of the neuromodulation system or at least one of patient's health metrics (e.g. physiological data before, while or after receiving electrical stimulation therapy, such as blood pressure, heartrate, SpO₂, movement, number of steps, etc.). It may comprise a space time programmer (STP) for e.g. programming spatial and temporal parameters of the stimulation.

In one embodiment, the second user input terminal is capable to receive data from the intermediate computing device or from the first user input terminal display data, receive input from the user (e.g. clinician or therapist), send it back to the intermediate computing device or the first user input terminal. In other words, second user input terminal can receive, display, process and/or resend data. In other words, the second user input terminal can be embodied such that it is possible to receive inter alia but not limited to stimulation parameters, patient data and the like, check and/or reprogram the stimulation settings and send it back to e.g. the intermediate computing device/controller.

Also, the first and/or the second user input terminal by be capable to send and receive data to or from a remote database.

In particular, the first and/or the second user input terminal may comprise a display and/or a graphical user interface.

The first and the second user input terminal can be embodied as applications installed on a mobile device that communicate with the intermediate computing device/ controller. They are used by the patient and the treating physician or physiotherapist, respectively, to provide inputs to the intermediate computing device/controller.

The first user input terminal and/or the second user input terminal can allow adjusting the stimulation parameters of a task, while the task (i.e. a stimulation program aimed at addressing a specific neurological function, such as locomotion) is running. This enables the user to tune the stimulation without having to start and stop the task, which would be very cumbersome and clinically undesired at the start of the rehabilitation training, when all stimulation partitures are developed and tuned.

In a further embodiment, the system further comprises a telemetry module and/or Bluetooth module and/or communication module and/or wireless network enabling connection between the medical device, the intermediate computing device, the first user input module and/or the second user input module. This may advantageously enable wireless and/or remote control and/or programming of the stimulation. For instance, the second user input terminal and its user can be remote from the first user input terminal.

In one embodiment, the connection between the medical device and the intermediate computing device is a bidirectional connection. In particular, the connection may be a wireless or cable-bound connection. Also, this connection may be a constant connection. This may enable continuous data transfer between the medical device and the intermediate computing device. For instance, the medical device can provide the intermediate computing device with patient data (sensed by a linked sensor), while the intermediate computing device provides the medical device with neuromodulation control data. This may be advantageous for settings where the stimulation program provided by the intermediate computing device shall be adapted to data provided by the medical device (e.g. patient data, in particular patient-feedback data).

Further, it is generally possible that the first user input terminal and/or the second user input terminal and/or the intermediate computing device are connected by a wireless connection. This has the advantage that the first user input terminal and/or the second user input terminal can be remote from the intermediate computing device. The wireless connection may be any remote telemetry techniques known in the art,
In one embodiment, the first user input terminal and/or the second user input terminal are remote from the intermediate computing device and/or the medical device. This has the advantage that the user of the first user input terminal can be remote from the user of the second user input terminal, and the user of the fist user input terminal and/or the user of the second user input terminal can be remote from the intermediate computing device and/or the medical device. For instance, a patient equipped with the first user input terminal, the medical device, and the intermediate computing device can be at home and thus remote from the second user input terminal (which may be e.g. placed in a hospital or laboratory).

In one embodiment, the system is configured and arranged for transferring data from the first user input terminal to the second user input terminal or vice versa, the first user input terminal to the intermediate computing device or vice versa, the second user input terminal to the intermediate computing device and/or the intermediate computing device to the medical device and/or vice versa. In particular, this may enable selective data transfer between respective transmitting devices/ terminals and/or receiving devices/terminals without transferring data to non-targeted devices/terminals.

In a further embodiment, the intermediate computing device comprises an applications module, wherein the first user input terminal and/or the second user input terminal are configured and arranged for accessing and/or controlling the applications module, in particular remotely accessing and/or controlling the applications module. In particular, the intermediate computing device may present information from the applications module to a user, e.g. via a display of the fist user input terminal and/or e.g. via a display of the second user input terminal, where the information includes a plurality of instructions for the medical device. In particular, remote access and control of the applications module may enable user-friendly and uncomplicated application of the system.

In a further embodiment, the first user input terminal and/or the second user input terminal comprise a display, wherein the intermediate computing device presents information from the applications module to a user via the display of the first user input terminal or the second user input terminal. This may be advantageous by enabling user-friendly application of the system, as e.g. patient data, programming options and/or instructions to be selected may be clearly arranged on the display. Thus, understanding and handling of the system is enabled.

In a further embodiment the first user input terminal and/or the second user input terminal comprise a user interface configured and arranged for receiving input from a user. In particular, the user interface may be a graphical user interface, or the display may present a graphical user interface that enables remote control of features of the intermediate computing device. This may enable easy and user-friendly application of the system.

Also, the intermediate computing device may be configured and arranged for receiving an indication of an input from a user, especially an indication of a selected instruction from a plurality of instructions for the medical device, and transmit the selected instruction to the medical device. In other words, the user input terminal may provide a plurality of possible instructions for instruction of the medical device to a user, and the user can select desired instructions which are transmitted to the intermediate computing device.

In particular, the selected instruction may include at least a request for diagnostic data from the medical device, patient data stored in the medical device or an input therapy parameter for programming into the implantable medical device. For instance, diagnostic data may be or may comprise neurological signals from the patient (e.g. sensed by a connected electrode), patient data may comprise physiological and/or pathological data, input therapy parameter may comprise starting and/or stopping and/or ramping up and/or ramping town stimulation for a task and/or configuration of stimulation parameters (electrode configuration, pulse width, amplitude, frequency, electrode configuration). In other words, a user may select at least one instruction, and the medical device operates according to the selected instruction, after the selected instructions is transmitted from the intermediate computing device to the medical device.

Further, the invention relates to a method for planning neuromodulation comprising:
- remotely accessing and/or controlling an intermediate computing device via a first user input terminal and/or a second user input terminal,
- providing user input to indicate a selected instruction from a plurality of instructions via a user interface of the first user input terminal and/or second user input terminal, wherein the intermediate computing device is configured to communicate the selected instruction to a medical device,
wherein the method further comprises the step of configuring and arranging a connection managing module for:
- allowing only unidirectional connection and/or unidirectional data transfer between the intermediate computing device and the first user input terminal, the intermediate computing device and the second user input terminal and/or the first user input terminal and the second user input terminal,
- providing only connection and/or data transfer between the intermediate computing device and the first user input terminal and/or the intermediate computing device and the second user input terminal when there is no connection and/or data transfer between the first user input terminal and the second user input terminal.

The method can be performed with the above-described system.

Further described is a method for planning neuromodulation, comprising:
a) with a first user input terminal, accessing, in particular remotely accessing, an applications module of an intermediate computing device;
b) presenting information from the applications module to a user via the first user input terminal;
c) transmitting data from the first user input terminal to a second user input terminal;
d) updating neuromodulation control data on the second user input terminal;
e) transfer updated neuromodulation control data from the second user input terminal to the intermediate computing device;
f) updating installed control commands on the first user input terminal by means of the updated neuromodulation control data;
g) using updated installed control commands for programming and/or controlling the intermediate computing device;
wherein the method further comprises the step of configuring and arranging a connection managing module for:
h) allowing only unidirectional connection and/or unidirectional data transfer between the intermediate computing device and the first user input terminal, the intermediate computing device and the second user input terminal and/or the first user input terminal and the second user input terminal,
i) providing only connection and/or data transfer between the intermediate computing device and the first user input terminal and/or the intermediate computing device and the second user input terminal when there is no connection and/or data transfer between the first user input terminal and the second user input terminal.

The control commands can also be understood as input therapy parameter.

As in c), the first user input terminal and the second user input terminal are connected for unidirectional data transfer from the first user input terminal to the second user input terminal while both the first user input terminal and the second user input terminal are not connected to the intermediate computing device, incidental overwriting of neuromodulation control data is avoided.

As in step d) there is no connection and/or data transfer between the first user input terminal, the second user input terminal and/or the intermediate computing device, incidental control commands and/or overwriting of neuromodulation control data provided is avoided.

As in step e) the second user input terminal and the intermediate computing device are connected and/or data are transferred unidirectionally from the second user input terminal to the intermediate computing device while the first user input terminal and the intermediate computing device and the first user input terminal and the second user input terminal are not connected, incidental control commands and/or overwriting of neuromodulation control data provided from the first user input terminal is avoided.

As in step f) the second user input terminal and the first user input terminal are connected and/or data are transferred unidirectionally from the second user input terminal to the first user input terminal while both the first user input terminal and the second user input terminal are not connected to the intermediate computing, interference of transferred data with neuromodulation control commands stored in the intermediate computing device is avoided.

As in step g), the first user input terminal and the intermediate computing device are connected and/or data are transferred unidirectionally from the first user input terminal to the intermediate computing device while the second user input terminal and the intermediate computing device and the second user input terminal and the first user input terminal are not connected, data interference is avoided. Also, as pre-installed control commands are presented the user, this enables keeping the handling of the system by the user (e.g. patient) simple and increases usability safety.

In particular, the methods described herein enable avoiding interference of data and/or signals transmitted between one user input terminal and the other user input terminal and/or the intermediate computing device. Therefore, controlled and secure transmission of data and/or signals at all time while the system is operating is enabled. Further, encryption of data may be enabled. Also, the system may enable saving energy and/or power and thus allow prolonging battery life time.

Further details and advantages of the present invention shall now be disclosed in connection with the drawings.

It is shown in:
- Fig. 1: a general layout of an embodiment of the system 10 according to the present invention;
- Fig. 2: a schematical illustration of a method performed with the system 10 disclosed in Fig. 1;
- Fig. 3: a further layout of the system 10, with unidirectional connection and/or data transfer between the fist user input terminal 18 and the controller 14;
- Fig. 4:: a further layout of the system 10, with unidirectional connection and/or data transfer between the fist user input terminal 18 and the second user input terminal;
- Fig. 5:: a further layout of the system 10;
- Fig. 6:: a further layout of the system 10, with unidirectional connection and/or data transfer between the second user input terminal 16 and the controller 14;
- Fig. 7:: a further layout of the system 10, with unidirectional connection and/or data transfer between the second user input terminal 16 and the first user input terminal 18; and
- Fig. 8:: a further layout of the system 10, with unidirectional connection and/or data transfer between the first user input terminal 18 and the controller 14.

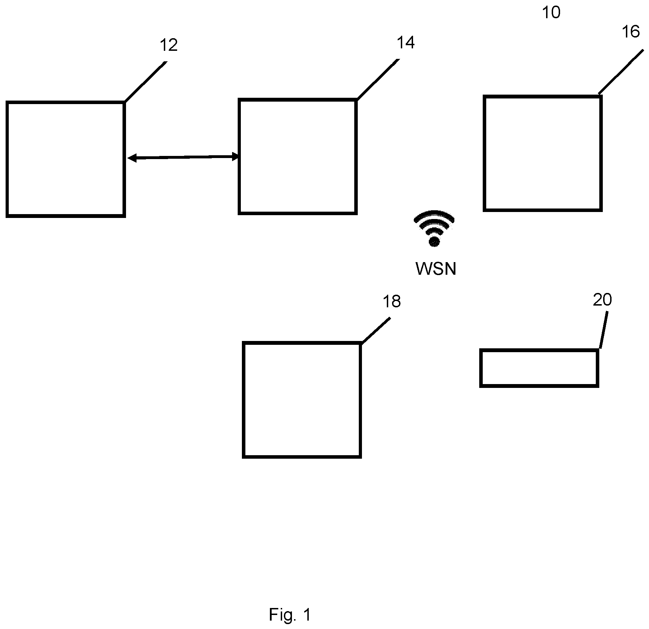

**Fig. 1** shows a general layout of an embodiment of the system 10 according to the present invention.

The system 10 comprises a medical device 12.

In this embodiment, the medical device 12 is an implantable pulse generator 12 which is implanted subcutaneously in a patient.

The system 10 further comprises an intermediate computing device 14.

In this embodiment, the intermediate computing device 14 is a controller 14, in particular a motion controller 14.

In this embodiment, the controller 14 is implanted in the patient.

In this embodiment, the controller 14 comprises an applications module.

The system 10 further comprises a first user input terminal 18.

In this embodiment, the first user input terminal 18 is a programmer 18, in particular a patient programmer 18.

In this embodiment, the first user input terminal 18 comprises a display.

Also, the first user input terminal 18 comprises a user interface, in particular a graphical user interface.

Also, the system 10 comprises a second user input terminal 16.

In this embodiment, the second user input terminal 16 is a programmer 16, in particular a clinician programmer 16.

In this embodiment, the second user input terminal 16 comprises a display.

Also, the second user input terminal 16 comprises a user interface, in particular a graphical user interface.

Alternatively, only the first user input terminal 18 or the second user input terminal 16 could comprise a display and/or a graphical user interface.

In this embodiment, the second user input terminal 16 is remote from the controller 14 and/or the implantable pulse generator 12.

Also, in general, the first user input terminal 18 can be remote from the controller 14 and/or the implantable pulse generator 12.

Also, the system 10 comprises a connection managing module 20.

In an alternative embodiment, the system 10 can comprise more than one connection managing module 20.

In this embodiment, the implantable pulse generator 12 and the controller 14 are connected.

The connection between the implantable pulse generator 12 and the controller 14 is a bidirectional connection.

The connection between the implantable pulse generator 12 and the controller 14 is a permanent and cable-bound connection.

In an alternative embodiment, the connection could be a wireless connection, e.g. a telemetry connection, wireless network connection, etc.

In general, also a unidirectional connection could be possible.

The first user input terminal 18 and the second user input terminal 16 are at least temporarily connected, in particular for data transfer.

Also, the first user input terminal 18 and the controller 14 are at least temporally connected, in particular for data transfer.

Also, the second user input terminal 16 and the controller 14 are at least temporally connected, in particular for data transfer.

The connection between the first user input terminal 18 and the controller 14, the first user input terminal 18 and the second user input terminal 16 and/or the controller 14 and the second user input terminal 16 is a wireless connection, in particular a connection via a wireless network WSN.

Alternatively, or additionally, the connection between the implanted pulse generator 12, the controller 14, the first user input terminal 18, the second user input terminal 16 and/or the connection managing module 20 can be enabled by a telemetry module and/or Bluetooth module and/or communication module.

In this embodiment, the connection managing module 20 allows only unidirectional connection and/or unidirectional data transfer between the controller 14 and the first user input terminal 18, the controller 14 and the second user input terminal 16 and/or the first user input terminal 18 and the second user input terminal 16.

In other words, the connection managing module can block bidirectional connection and/or data transfer between the controller 14 and the first user input terminal 18, the controller 14 and the second user input terminal 16 and/or the first user input terminal 18 and the second user input terminal 16.

Further, in this embodiment, the connection managing module 20 provides only connection and/or data transfer between the controller 14 and the first user input terminal 18 and/or the controller 14 and the second user input terminal 16 when there is no connection and/or data transfer between the first user input terminal 18 and the second user input terminal 16.

In other words, the connection managing module 20 can block connection and/or data transfer between the controller 14 and the first user input terminal 18 and/or the controller 14 and the second user input terminal 16 when there is already a connection and/or data transfer between the first user input terminal 18 and the second user input terminal 16.

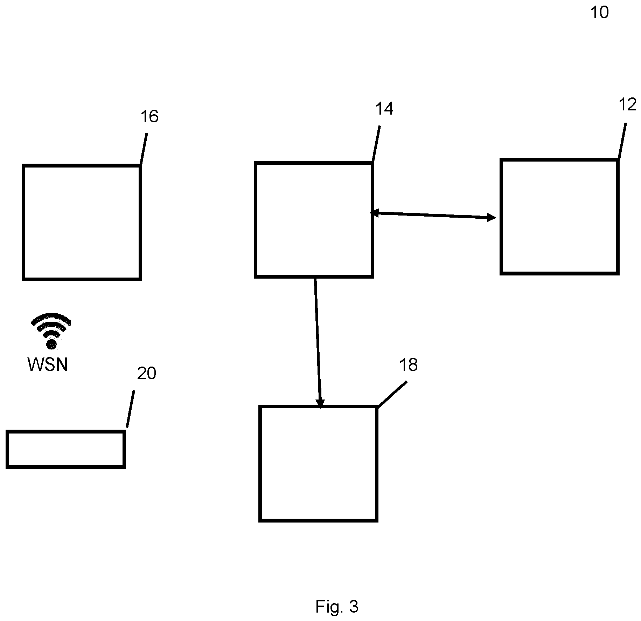

In other words, the connection managing module 20 can switch connection and/or data transfer between the different units/devices/terminals of the system 10, cf. Figs. 3-8.

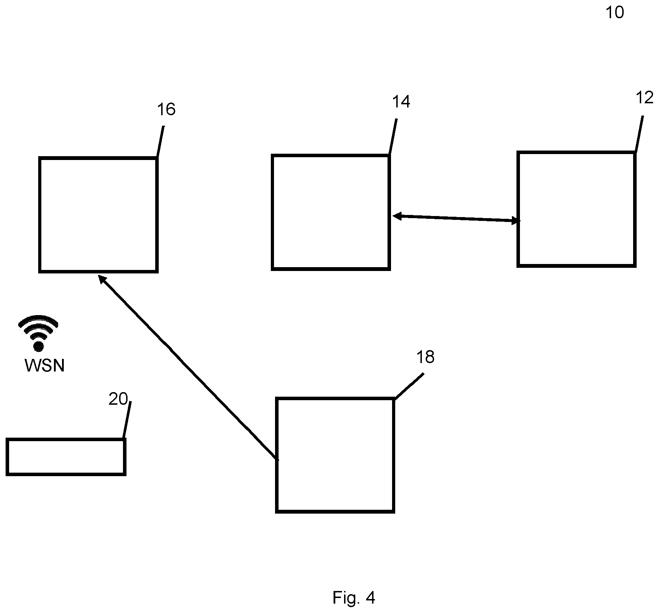

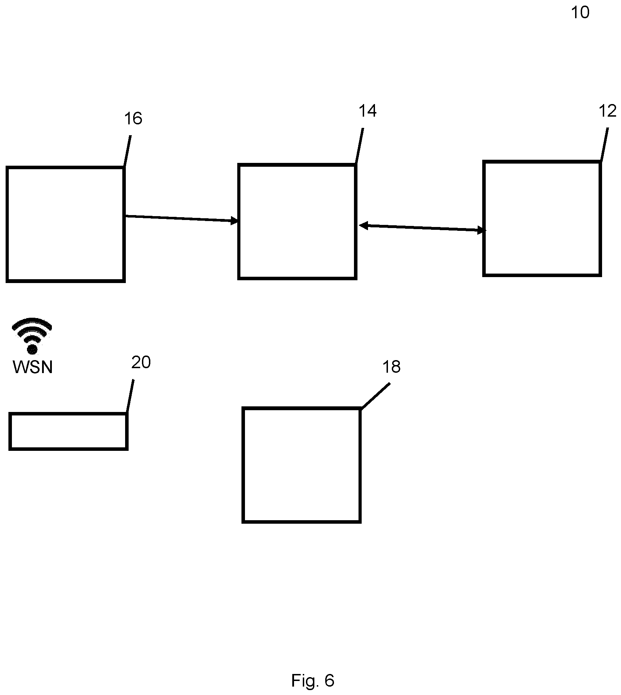

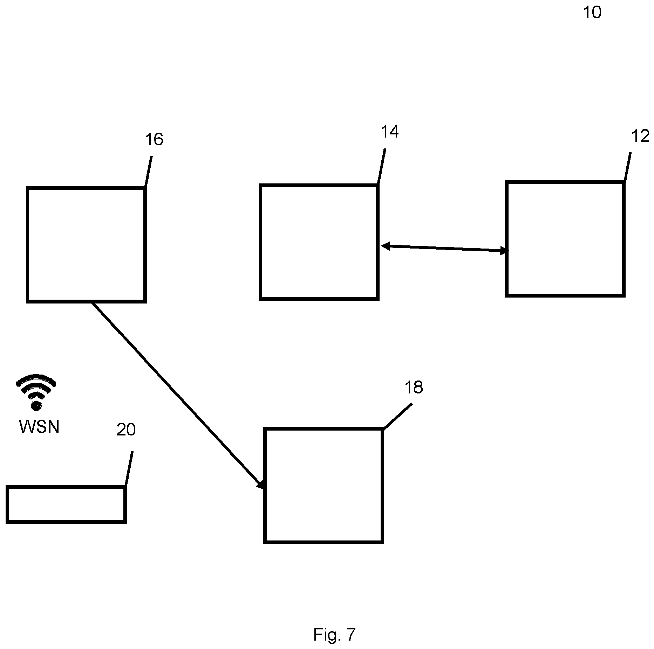

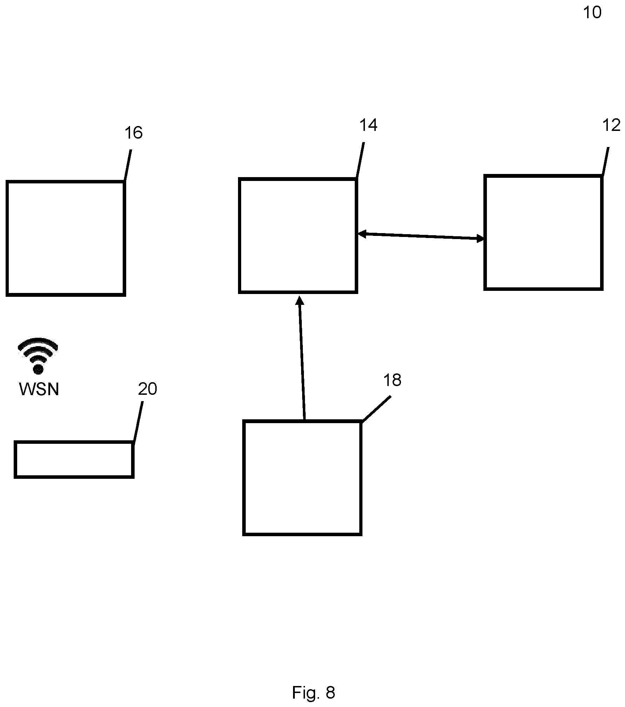

Not explicitly shown in Fig. 1 is that the system 10 enables transfer of data from the first user input terminal 18 to the second user input terminal 16 or vice versa (cf. Figs. 4 and 7), the first user input terminal 18 to the controller 14 or vice versa (cf. Figs. 3 and 8), the second user input terminal 16 to the controller 14 or vice versa (cf. e.g. Fig. 6 ) and/or the controller 14 to the medical device 12 and/or vice versa (Figs. 3-8).

The first user input terminal 18 and the second user input terminal 16 access and/or control the applications module, in particular remotely access and/or control the applications module.

Not shown in Fig. 1 is that the controller 14 presents information from the applications module to a user (e.g. a patient) via the display of the first user input terminal 18, in particular input therapy parameters for section may me presented to the user.

Additionally, or alternatively, the controller 14 can present information from the applications module to a user (e.g. a clinician) via the display of the second user input terminal 16, in particular stimulation parameters may be presented.

Not shown in Fig. 1 is that the user interface of the first user input terminal 18 can receive input from a user.

The user input can then be processed by the first user input terminal 18 and/or transmitted to the controller 14 or the second user input terminal 16. In particular processed data can be transmitted to the controller 14 or the second user input terminal 16.

Also not shown in Fig. 1 is that the user interface of the second user input terminal 16 can receive input from a user.

The user input can then be processed by the second user input terminal 16 and/or transmitted to the controller 14 or the first user input terminal 18. In particular processed data can be transmitted to the controller 14 or the first user input terminal 18.

Further not shown is that, in particular based on the user input, in particular the processed user input, the controller 14 receives an indication of the input from a user, especially an indication of a selected instruction from a plurality of instructions for the implantable pulse generator 12, and transmits the selected instruction to the implantable pulse generator 12

The selected instruction can comprise at least one of a request for diagnostic data from the implantable pulse generator 12, e.g. measured neuroelectric activity, patient data stored within the implantable pulse generator or at least one input therapy parameter for programming into the implantable pulse generator 12.

The implantable pulse generator 12 can provide the controller 14 with the diagnostic data or with patient data, or can provide stimulation based on the input therapy parameters selected.

Thus, in other words, system 10 can perform the the method according to claim 11.

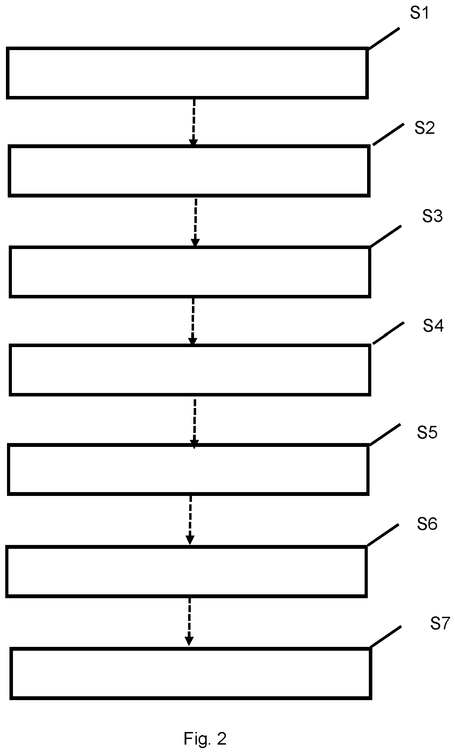

Also, the system 10 can perform the method as disclosed in Fig. 2.

The method comprises at least the steps S1-S7.

In a first step S1, by means of the first user input terminal 18 the applications module of the controller 14 can be accessed, in particular remotely accessed.

In other words, data relating to or of the implantable pulse generator 12 and/or the controller 14 can be accessed. In other words, the applications module and (indirectly) the implantable pulse generator 12 can be accessed.

In step S1, the controller 14 is connected to the first user input terminal 18 and data are transferred unidirectionally from the controller 14 to the first user input terminal 18, cf. Fig. 3.

In a second step S2, the information, in particular accessed data from the applications module, can be presented to a user via the first user input terminal 18, in particular the display of the first user input terminal 18.

In a third step S3, the data can be transmitted from the first user input terminal 18 to the second user input terminal 16.

In step S3, connection between the first user input terminal 18 and the second user input terminal 16 and/or unidirectional data transfer from the first user input terminal 18 to the second user input terminal 16 is enabled, while connection and/or data transfer between the first user input terminal 18 and the controller 14 and the second user input terminal 16 and the controller is not enabled, cf. Fig. 4.

In a fourth step S4, neuromodulation control data and/or stimulation parameters can be updated on the second user input terminal 16, in particular based on the data transmitted from the first user input terminal 18.

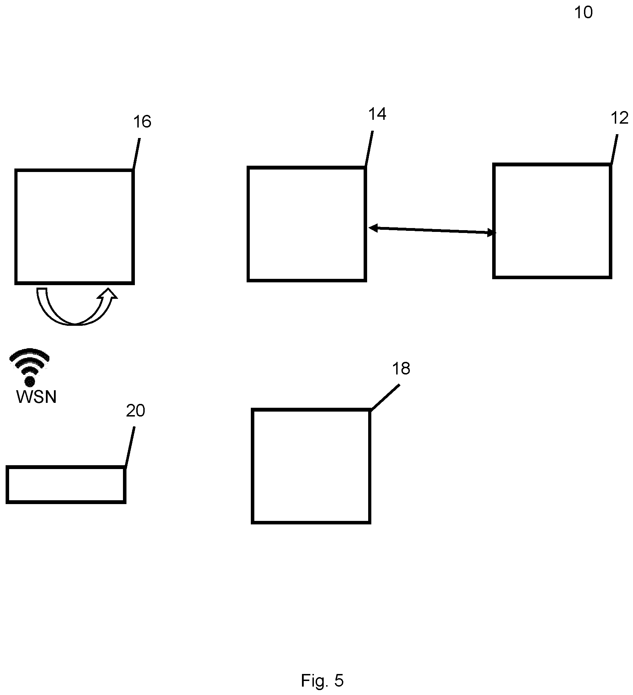

In step S4, no connection and/or data transfer between the second user input terminal 16 and the first user input terminal 18 or the controller 14 is enabled, cf. Fig. 5.

In a fifth step S5, the updated neuromodulation control data and/or stimulation parameters can be transferred from the second user input terminal 16 to the controller 14.

In step S5, connection between the second user input terminal 16 and the controller 14 and/or unidirectional data transfer from the second user input terminal 16 to the controller 14 is enabled, while connection and/or data transfer between the first user input terminal 18 and the controller 14 and the second user input terminal 16 and the first user input terminal 18 is not enabled, cf. Fig. 6.

In a sixth step S6, the installed control commands (input therapy parameters) on the first user input terminal 18 can be updated by means of the updated neuromodulation control data.

In step S6, connection between the second user input terminal 16 and the first user input terminal 18 and/or unidirectional data transfer from the second user input terminal 16 to the first user input terminal 18 is enabled, while connection and/or data transfer between the first user input terminal 18 and the controller 14 and the second user input terminal 16 and the controller 14 is not enabled, cf. Fig. 7.

In a seventh step S7, the updated installed control commands (input therapy parameter(s)) can be used for programming and/or controlling the controller 14.

In step S7, connection between the first user input terminal 18 and the controller 14 and/or unidirectional data transfer from the first user input terminal 18 to controller 14 is enabled, while connection and/or data transfer between the second user input terminal 16 and the controller 14 and the second user input terminal 16 and the first user input terminal 18 is not enabled, cf. Fig. 8.

Thus, the method allows only unidirectional connection and/or unidirectional data transfer between the controller 14 and the first user input terminal 18, the controller 14) and the second user input terminal 16 and/or the first user input terminal 18 and the second user input terminal 16.

Further, the method provides only connection and/or data transfer between the controller 14 and the first user input terminal 18 and/or the controller 14 and the second user input terminal 16 when there is no connection and/or data transfer between the first user input terminal 18 and the second user input terminal 16.

Fig. 3 shows a further layout of the system 10, with unidirectional connection and/or data transfer between the fist user input terminal 18 and the controller 14.

The structural and functional features of the system 10 are described in detail in Fig. 1.

In this embodiment, a unidirectional connection between the controller 14 and the first user input terminal 18 and/or unidirectional data transfer from the controller 14 to the first user input terminal 18 is enabled.

In particular, the application module of the controller 14 is remotely accessed by means of the first user input terminal 18 and data are got out of the medical device 12.

**Fig. 4** shows a further layout of the system 10, with unidirectional connection and/or data transfer between the fist user input terminal 18 and the second user input terminal 16.

The structural and functional features of the system 10 are described in detail in Fig. 1.

In this embodiment, unidirectional connection between the first user input terminal 18 and the second user input terminal 16 and/or unidirectional data transfer from the first user input terminal 18 to the second user input terminal is enabled, while connection and/or data transfer between the first user input terminal 18 and the controller 14 and the second user input terminal 16 and the controller is not enabled.

In particular, the connection enables that data are transmitted from the first user input terminal 18 to the second user input terminal 16.

**Fig. 5** shows a further layout of the system 10.

The structural and functional features of the system 10 are described in detail in Fig. 1.

In particular, no connection and/or data transfer between the second user input terminal 16 and the first user input terminal 18 and/or the controller 14 is enabled while neuromodulation control data and/or stimulation parameters are updated on the second user input terminal 16.

**Fig. 6** shows a further layout of an embodiment of the system 10, with unidirectional connection and/or data transfer between the second user input terminal 16 and the controller 14.

The structural and functional features of the system 10 are described in detail in Fig. 1.

In this embodiment, unidirectional connection between the second user input terminal 16 and the controller 14 and/or unidirectional data transfer from the second user input terminal 16 to the controller 14 is enabled, while connection and/or data transfer between the first user input terminal 18 and the controller 14 and the second user input terminal 16 and the first user input terminal 18 is not enabled.

In particular, through this connection, the controller 14 can be provided with updated neuromodulation control data.

**Fig. 7** shows a further layout of an embodiment of the system 10, with unidirectional connection and/or data transfer between the second user input terminal 16 and the first user input terminal 18.

The structural and functional features of the system 10 are described in detail in Fig. 1.

In this embodiment, unidirectional connection between the second user input terminal 16 and the first user input terminal 18 and/or unidirectional data transfer from the second user input terminal 16 to the first user input terminal 18 is enabled, while connection and/or data transfer between the first user input terminal 18 and the controller 14 and the second user input terminal 16 and the controller 14 is not enabled.

In particular, the connection can enable updating installed control commands (input therapy parameters) on the first user input terminal 18 by means of updated neuromodulation control data provided by the second user input terminal 16.

**Fig. 8** shows a further layout of an embodiment of the system 10, with unidirectional connection and/or data transfer between the first user input terminal 18 and the controller 14.

The structural and functional features of the system 10 are described in detail in Fig. 1.

In this embodiment, connection between the first user input terminal 18 and the controller 14 and/or unidirectional data transfer from the first user input terminal 18 to controller 14 is enabled, while connection and/or data transfer between the second user input terminal 16 and the controller 14 and the second user input terminal 16 and the first user input terminal 18 is not enabled.

The connection can enable programming and/or controlling the controller 14 by means of updated installed control commands (input therapy parameter(s)) (cf. Fig. 7).

### References

- 10: system
- 12: medical device/ implantable pulse generator
- 14: intermediate computing device/controller
- 16: second user input terminal
- 18: first user input terminal
- 20: connection managing module

## Claims

1. A system (10) for planning and/or controlling neuromodulation, at least comprising:
a medical device (12),
an intermediate computing device (14),
a first user input terminal (18),
a second user input terminal (16),
wherein the medical device (12), the intermediate computing device (14), the first user input terminal (18) and/or the second user input terminal (16) are at least temporarily connected,
wherein the system further comprises at least one connection managing module (20), wherein the at least one connection managing module (20) is configured and arranged for
- allowing only unidirectional connection and/or unidirectional data transfer between the intermediate computing device (14) and the first user input terminal (18), the intermediate computing device (14) and the second user input terminal (16) and/or the first user input terminal (18) and the second user input terminal (16),
- providing only connection and/or data transfer between the intermediate computing device (14) and the first user input terminal (18) and/or the intermediate computing device (14) and the second user input terminal (16) when there is no connection and/or data transfer between the first user input terminal (18) and the second user input terminal (16).

2. The system (10) according to claim 1, **characterized in that** the system (10) further comprises a telemetry module and/or Bluetooth module and/or communication module and/or wireless network enabling connection between the medical device, the intermediate computing device (14), the first user input module (18) and/or the second user input module (16).

3. The system (10) according to claim 1 or claim 2, **characterized in that** the connection between the medical device (12) and the intermediate computing device (14) is a bidirectional connection.

4. The system (10) according to claim 1, claim 2 or claim 3, **characterized in that** the system (10) is configured and arranged for transferring data from the first user input terminal (18) to the second user input terminal (16) or vice versa, the first user input terminal (18) to the intermediate computing device (14) or vice versa, the second user input terminal (16) to the intermediate computing device (14) and/or the intermediate computing device (14) to the medical device (12) and/or vice versa.

5. The system (10) according to claim 1, claim 2, claim 3 or claim 4, **characterized in that** the first user input terminal (18) and/or the second user input terminal (16) are remote from the intermediate computing device (14) and/or the medical device (12).

6. The system (10) according to one of claims 1 to 5, **characterized in that** the intermediate computing device (14) comprises an applications module, wherein the first user input terminal (18) and/or the second user input terminal (16) are configured and arranged for accessing and/or controlling the applications module, in particular remotely accessing and/or controlling the applications module.

7. The system (10) according to one of claims 1 to 6, **characterized in that** the first user input terminal (18) and/or the second user input terminal (16) comprise a display, wherein the intermediate computing device (14) presents information from the applications module to a user via the display of the first user input terminal (18) or the second user input terminal (16).

8. The system (10) according to one of claims 1 to 7, **characterized in that** the first user input terminal (18) and/or the second user input terminal (16) comprise a user interface configured and arranged for receiving input from a user.

9. The system (10) according to one of the preceding claims, **characterized in that** the intermediate computing device (14) is configured and arranged for receiving an indication of an input from a user, especially an indication of a selected instruction from a plurality of instructions for the medical device (12), and transmit the selected instruction to the medical device (12).

10. The system (10) according to claim 9, **characterized in that** the selected instruction comprises at least one of a request for diagnostic data from the medical device, patient data stored within the medical device (12) or an input therapy parameter for programming into the medical device (12).

11. A method for planning neuromodulation, comprising
- remotely accessing and/or controlling an intermediate computing device (14) via a first user input terminal (18) and/or a second user input terminal (16),
- providing user input to indicate a selected instruction from a plurality of instructions via a user interface of the first user input terminal (18) and/or second user input terminal (16), wherein the intermediate computing device (14) is configured to communicate the selected instruction to a medical device (12),
wherein the method further comprises the step of configuring and arranging a connection managing module (20) for:
- allowing only unidirectional connection and/or unidirectional data transfer between the intermediate computing device (14) and the first user input terminal (18), the intermediate computing device (14) and the second user input terminal (16) and/or the first user input terminal (18) and the second user input terminal (16),
- providing only connection and/or data transfer between the intermediate computing device (14) and the first user input terminal (18) and/or the intermediate computing device (14) and the second user input terminal (16) when there is no connection and/or data transfer between the first user input terminal (18) and the second user input terminal (16).

12. The method according to claim 11, comprising:
a) accessing, in particular remotely accessing, the applications module of the intermediate computing device (14) with the first user input terminal (18);
b) presenting information from the applications module to a user via the first user input terminal (18);
c) transmitting data from the first user input terminal (18) to the second user input terminal (16);
d) updating neuromodulation control data on the second user input terminal (16);
e) transfer updated neuromodulation control data from the second user input terminal (16) to the intermediate computing device (14);
f) updating installed control commands on the first user input terminal (18) by means of the updated neuromodulation control data;
g) using updated installed control commands for programming and/or controlling the intermediate computing device (14).

## Patentansprüche

1. System (10) zum Planen und/oder Steuern einer Neuromodulation, mindestens umfassend:
eine medizinisches Vorrichtung (12),
eine Zwischenrechenvorrichtung (14),
ein erstes Benutzereingabeterminal (18),
ein zweites Benutzereingabeterminal (16),
wobei die medizinische Vorrichtung (12), die Zwischenrechenvorrichtung (14), das erste Benutzereingabeterminal (18) und/oder das zweite Benutzereingabeterminal (16) mindestens zeitweise verbunden sind,
wobei das System ferner mindestens ein Verbindungsverwaltungsmodul (20) umfasst, wobei das mindestens eine Verbindungsverwaltungsmodul (20) konfiguriert und angeordnet ist zum
- Zulassen nur einer unidirektionalen Verbindung und/oder einer unidirektionalen Datenübertragung zwischen der Zwischenrechenvorrichtung (14) und dem ersten Benutzereingabeterminal (18), der Zwischenrechenvorrichtung (14) und dem zweiten Benutzereingabeterminal (16) und/oder dem ersten Benutzereingabeterminal (18) und dem zweiten Benutzereingabeterminal (16),
- Bereitstellen nur einer Verbindung und/oder einer Datenübertragung zwischen der Zwischenrechenvorrichtung (14) und dem ersten Benutzereingabeterminal (18) und/oder der Zwischenrechenvorrichtung (14) und dem zweiten Benutzereingabeterminal (16), wenn keine Verbindung und/oder keine Datenübertragung zwischen dem ersten Benutzereingabeterminal (18) und dem zweiten Benutzereingabeterminal (16) besteht.

2. System (10) nach Anspruch 1, **dadurch gekennzeichnet, dass** das System (10) ferner ein Telemetriemodul und/oder ein Bluetooth-Modul und/oder ein Kommunikationsmodul und/oder ein drahtloses Netzwerk umfasst, das die Verbindung zwischen der medizinischen Vorrichtung, der Zwischenrechenvorrichtung (14), dem ersten Benutzereingabemodul (18) und/oder dem zweiten Benutzereingabemodul (16) ermöglicht.

3. System (10) nach Anspruch 1 oder 2, **dadurch gekennzeichnet, dass** die Verbindung zwischen der medizinischen Vorrichtung (12) und der Zwischenrechenvorrichtung (14) eine bidirektionale Verbindung ist.

4. System (10) nach Anspruch 1, 2 oder 3, **dadurch gekennzeichnet, dass** das System (10) zum Übertragen von Daten von dem ersten Benutzereingabeterminal (18) zu dem zweiten Benutzereingabeterminal (16) oder umgekehrt, dem ersten Benutzereingabeterminal (18) zu der Zwischenrechenvorrichtung (14) oder umgekehrt, dem zweiten Benutzereingabeterminal (16) zu der Zwischenrechenvorrichtung (14) und/oder der Zwischenrechenvorrichtung (14) zu der medizinischen Vorrichtung (12) und/oder umgekehrt konfiguriert und angeordnet ist.

5. System (10) nach Anspruch 1, 2, 3 oder 4, **dadurch gekennzeichnet, dass** das erste Benutzereingabeterminal (18) und/oder das zweite Benutzereingabeterminal (16) von der Zwischenrechenvorrichtung (14) und/oder der medizinischen Vorrichtung (12) entfernt sind.

6. System (10) nach einem der Ansprüche 1 bis 5,
**dadurch gekennzeichnet, dass** die Zwischenrechenvorrichtung (14) ein Anwendungsmodul umfasst, wobei das erste Benutzereingabeterminal (18) und/oder das zweite Benutzereingabeterminal (16) zum Zugreifen auf das Anwendungsmodul und/oder Steuern von diesem, insbesondere entferntes Zugreifen auf das Anwendungsmodul und/oder Steuern von diesem, konfiguriert und angeordnet ist.

7. System (10) nach einem der Ansprüche 1 bis 6,
**dadurch gekennzeichnet, dass** das erste Benutzereingabeterminal (18) und/oder das zweite Benutzereingabeterminal (16) eine Anzeige umfassen, wobei die Zwischenrechenvorrichtung (14) einem Benutzer Informationen aus dem Anwendungsmodul über die Anzeige des ersten Benutzereingabeterminals (18) oder des zweiten Benutzereingabeterminals (16) präsentiert.

8. System (10) nach einem der Ansprüche 1 bis 7,
**dadurch gekennzeichnet, dass** das erste Benutzereingabeterminal (18) und/oder das zweite Benutzereingabeterminal (16) eine Benutzerschnittstelle umfassen, die zum Empfangen von Eingaben von einem Benutzer konfiguriert und angeordnet ist.

9. System (10) nach einem der vorstehenden Ansprüche,
**dadurch gekennzeichnet, dass** die Zwischenrechenvorrichtung (14) zum Empfangen einer Angabe einer Eingabe von einem Benutzer, insbesondere einer Angabe einer ausgewählten Anweisung aus einer Vielzahl von Anweisungen für die medizinische Vorrichtung (12), und Übertragen der ausgewählten Anweisung an die medizinische Vorrichtung (12) konfiguriert und angeordnet ist.

10. System (10) nach Anspruch 9, **dadurch gekennzeichnet, dass** die ausgewählte Anweisung mindestens eines von einer Anforderung von Diagnosedaten von der medizinischen Vorrichtung, Patientendaten, die innerhalb der medizinischen Vorrichtung (12) gespeichert sind, oder einem eingegebenen Therapieparameter zum Programmieren in die medizinischen Vorrichtung (12) umfasst.

11. Verfahren zum Planen der Neuromodulation, umfassend:
- entferntes Zugreifen auf und/oder Steuern einer Zwischenrechenvorrichtung (14) über ein erstes Benutzereingabeterminal (18) und/oder ein zweites Benutzereingabeterminal (16),
- Bereitstellen einer Benutzereingabe, um eine ausgewählte Anweisung aus einer Vielzahl von Anweisungen über eine Benutzerschnittstelle des ersten Benutzereingabeterminals (18) und/oder des zweiten Benutzereingabeterminals (16) anzugeben, wobei die Zwischenrechenvorrichtung (14) konfiguriert ist, um die ausgewählte Anweisung an eine medizinische Vorrichtung (12) zu kommunizieren,
wobei das Verfahren ferner den Schritt des Konfigurierens und Anordnens eines Verbindungsverwaltungsmoduls (20) umfasst zum:
- Zulassen nur der unidirektionalen Verbindung und/oder der unidirektionalen Datenübertragung zwischen der Zwischenrechenvorrichtung (14) und dem ersten Benutzereingabeterminal (18), der Zwischenrechenvorrichtung (14) und dem zweiten Benutzereingabeterminal (16) und/oder dem ersten Benutzereingabeterminal (18) und dem zweiten Benutzereingabeterminal (16),
- Bereitstellen nur der Verbindung und/oder einer Datenübertragung zwischen der Zwischenrechenvorrichtung (14) und dem ersten Benutzereingabeterminal (18) und/oder der Zwischenrechenvorrichtung (14) und dem zweiten Benutzereingabeterminal (16), wenn keine Verbindung und/oder keine Datenübertragung zwischen dem ersten Benutzereingabeterminal (18) und dem zweiten Benutzereingabeterminal (16) besteht.

12. Verfahren nach Anspruch 11, umfassend:
a) Zugreifen, insbesondere entferntes Zugreifen, auf das Anwendungsmodul der Zwischenrechenvorrichtung (14) mit dem ersten Benutzereingabeterminal (18);
b) Präsentieren von Informationen aus dem Anwendungsmodul an einen Benutzer über das erste Benutzereingabeterminal (18);
c) Übertragen von Daten von dem ersten Benutzereingabeterminal (18) zu dem zweiten Benutzereingabeterminal (16);
d) Aktualisieren der Neuromodulationssteuerdaten auf dem zweiten Benutzereingabeterminal (16);
e) Übertragen aktualisierter Neuromodulationssteuerdaten von dem zweiten Benutzereingabeterminal (16) an die Zwischenrechenvorrichtung (14);
f) Aktualisieren von installierten Steuerbefehlen auf dem ersten Benutzereingabeterminal (18) mittels der aktualisierten Neuromodulationssteuerdaten;
g) Verwenden von aktualisierten installierten Steuerbefehlen zum Programmieren und/oder Steuern der Zwischenrechenvorrichtung (14).

## Revendications

1. Système (10) de planification et/ou de commande de neuromodulation, comprenant au moins :
un dispositif médical (12),
un dispositif informatique intermédiaire (14),
un premier terminal d'entrée utilisateur (18),
un second terminal d'entrée utilisateur (16),
dans lequel le dispositif médical (12), le dispositif informatique intermédiaire (14), le premier terminal d'entrée utilisateur (18) et/ou le second terminal d'entrée utilisateur (16) sont au moins temporairement connectés,
dans lequel le système comprend en outre au moins un module de gestion de connexion (20), dans lequel l'au moins un module de gestion de connexion (20) est configuré et agencé pour
- permettre uniquement une connexion unidirectionnelle et/ou un transfert de données unidirectionnel entre le dispositif informatique intermédiaire (14) et le premier terminal d'entrée utilisateur (18), le dispositif informatique intermédiaire (14) et le second terminal d'entrée utilisateur (16) et/ou le premier terminal d'entrée utilisateur (18) et le second terminal d'entrée utilisateur (16),
- assurer uniquement une connexion et/ou un transfert de données entre le dispositif informatique intermédiaire (14) et le premier terminal d'entrée utilisateur (18) et/ou le dispositif informatique intermédiaire (14) et le second terminal d'entrée utilisateur (16) lorsqu'il n'y a pas de connexion et/ou de transfert de données entre le premier terminal d'entrée utilisateur (18) et le second terminal d'entrée utilisateur (16).

2. Système (10) selon la revendication 1, **caractérisé en ce que** le système (10) comprend en outre un module de télémétrie et/ou un module Bluetooth et/ou un module de communication et/ou un réseau sans fil permettant une connexion entre le dispositif médical, le dispositif informatique intermédiaire (14), le premier module d'entrée utilisateur (18) et/ou le second module d'entrée utilisateur (16).

3. Système (10) selon la revendication 1 ou la revendication 2, **caractérisé en ce que** la connexion entre le dispositif médical (12) et le dispositif informatique intermédiaire (14) est une connexion bidirectionnelle.

4. Système (10) selon la revendication 1, la revendication 2 ou la revendication 3, **caractérisé en ce que** le système (10) est configuré et agencé pour transférer des données du premier terminal d'entrée utilisateur (18) au second terminal d'entrée utilisateur (16) ou inversement, du premier terminal d'entrée utilisateur (18) au dispositif informatique intermédiaire (14) ou inversement, du second terminal d'entrée utilisateur (16) au dispositif informatique intermédiaire (14) et/ou du dispositif informatique intermédiaire (14) au dispositif médical (12) et/ou inversement.

5. Système (10) selon la revendication 1, la revendication 2, la revendication 3 ou la revendication 4, **caractérisé en ce que** le premier terminal d'entrée utilisateur (18) et/ou le second terminal d'entrée utilisateur (16) sont éloignés du dispositif informatique intermédiaire (14) et/ou du dispositif médical (12).

6. Système (10) selon l'une des revendications 1 à 5, **caractérisé en ce que** le dispositif informatique intermédiaire (14) comprend un module d'applications, dans lequel le premier terminal d'entrée utilisateur (18) et/ou le second terminal d'entrée utilisateur (16) sont configurés et agencés pour accéder au module d'applications et/ou le commander, en particulier accéder au module d'applications et/ou le commander à distance.

7. Système (10) selon l'une des revendications 1 à 6, **caractérisé en ce que** le premier terminal d'entrée utilisateur (18) et/ou le second terminal d'entrée utilisateur (16) comprennent un dispositif d'affichage, dans lequel le dispositif informatique intermédiaire (14) présente des informations provenant du module d'applications à un utilisateur par le biais du dispositif d'affichage du premier terminal d'entrée utilisateur (18) ou du second terminal d'entrée utilisateur (16).

8. Système (10) selon l'une des revendications 1 à 7, **caractérisé en ce que** le premier terminal d'entrée utilisateur (18) et/ou le second terminal d'entrée utilisateur (16) comprennent une interface utilisateur configurée et agencée pour recevoir une entrée provenant d'un utilisateur.

9. Système (10) selon l'une des revendications précédentes, **caractérisé en ce que** le dispositif informatique intermédiaire (14) est configuré et agencé pour recevoir une indication d'une entrée provenant d'un utilisateur, en particulier une indication d'une instruction choisie parmi une pluralité d'instructions pour le dispositif médical (12), et transmettre l'instruction choisie au dispositif médical (12).

10. Système (10) selon la revendication 9, **caractérisé en ce que** l'instruction choisie comprend au moins l'un parmi une demande de données de diagnostic provenant du dispositif médical, des données de patient stockées dans le dispositif médical (12) ou un paramètre de thérapie d'entrée en vue d'une programmation dans le dispositif médical (12).

11. Procédé de planification de neuromodulation, comprenant :
- l'accès à un dispositif informatique intermédiaire (14) et/ou sa commande, à distance, par le biais d'un premier terminal d'entrée utilisateur (18) et/ou d'un second terminal d'entrée utilisateur (16),
- la fourniture d'une entrée utilisateur pour indiquer une instruction choisie parmi une pluralité d'instructions par le biais d'une interface utilisateur du premier terminal d'entrée utilisateur (18) et/ou du second terminal d'entrée utilisateur (16), dans lequel le dispositif informatique intermédiaire (14) est configuré pour communiquer l'instruction choisie à un dispositif médical (12),
dans lequel le procédé comprend en outre l'étape consistant à configurer et à agencer un module de gestion de connexion (20) pour :
- permettre uniquement une connexion unidirectionnelle et/ou un transfert de données unidirectionnel entre le dispositif informatique intermédiaire (14) et le premier terminal d'entrée utilisateur (18), le dispositif informatique intermédiaire (14) et le second terminal d'entrée utilisateur (16) et/ou le premier terminal d'entrée utilisateur (18) et le second terminal d'entrée utilisateur (16),
- assurer uniquement une connexion et/ou un transfert de données entre le dispositif informatique intermédiaire (14) et le premier terminal d'entrée utilisateur (18) et/ou le dispositif informatique intermédiaire (14) et le second terminal d'entrée utilisateur (16) lorsqu'il n'y a pas de connexion et/ou de transfert de données entre le premier terminal d'entrée utilisateur (18) et le second terminal d'entrée utilisateur (16).

12. Procédé selon la revendication 11, comprenant :
a) l'accès, en particulier l'accès à distance, au module d'applications du dispositif informatique intermédiaire (14) avec le premier terminal d'entrée utilisateur (18) ;
b) la présentation d'informations provenant du module d'applications à un utilisateur par le biais du premier terminal d'entrée utilisateur (18) ;
c) la transmission de données du premier terminal d'entrée utilisateur (18) au second terminal d'entrée utilisateur (16) ;
d) la mise à jour de données de commande de neuromodulation sur le second terminal d'entrée de l'utilisateur (16) ;
e) le transfert de données de commande de neuromodulation mises à jour du second terminal d'entrée utilisateur (16) au dispositif informatique intermédiaire (14) ;
f) la mise à jour d'instructions installées sur le premier terminal d'entrée utilisateur (18) au moyen des données de commande de neuromodulation mises à jour;
g) l'utilisation d'instructions de commande installées mises à jour pour programmer et/ou commander le dispositif informatique intermédiaire (14).
